(19) Europäisches Patentamt
European Patent Office
Office européen des brevets

(11) Publication number: **0 146 219**
**A1**

(12) **EUROPEAN PATENT APPLICATION**

(21) Application number: 84306751.3

(22) Date of filing: 04.10.84

(51) Int. Cl.⁴: C 07 F 9/38, C 07 F 9/30, C 07 D 249/14, A 01 N 57/18

(30) Priority: 23.11.83 IL 70299

(43) Date of publication of application: 26.06.85 Bulletin 85/26

(84) Designated Contracting States: AT BE CH DE FR GB IT LI LU NL SE

(71) Applicant: Geshuri Laboratories Ltd., Industrial Zone, Tel-Mond (IL)

(72) Inventor: Bakel, Izhak, 177 Modein Street, Ramat Gan (IL)

(74) Representative: Ablewhite, Alan James et al, MARKS & CLERK 57/60 Lincoln's Inn Fields, London WC2A 3LS (GB)

(54) **Aminophosphonic & aminophosphinic acid derivatives processes for their preparation & herbicidal compositions containing them.**

(57) The invention provides 3-amino-1,2,4-triazole salts of aminophosphonic and aminophosphinic acids. The invention also provides phototoxic compositions containing these 3-amino-1,2,4-triazole salts and processes for the preparation thereof.

EP 0 146 219 A1

# AMINOPHOSPHONIC & AMINOPHOSPHINIC ACID DERIVATIVES. PROCESSES FOR THEIR PREPARATION & HERBICIDAL COMPOSITIONS CONTAINING THEM

The present invention relates to Aminophosphonic and Aminophosphinic acids and herbicidal compositions containing them. More specifically, the present invention relates to a whole series of newly discovered 3-amino-1,2,4-triazole derivative salts of such acids as N-Phosphonomethylglycine (NPMG), N-Phosphonomethyliminodiacetic acid (NPMIDA) and 2-amino-4-(methylphosphino) butanoic acid (phosphinothricine) which surprisingly exhibit pre- and post-emergent phytotoxic properties superior to the most effective derivatives of the above amino acids marketed to date. Moreover, the herbicidal salts described herein are advantageously employed to replace mechanical tillage in the preparation of a seedbed in no-tillage farming.

The compounds NPMIDA, Phosphinothricine and in particular NPMG are very effective post-emergent herbicides, however, because they are relatively insoluble in water and conventional organic solvents, they are not as readily amenable to commercial formulation as are their derivatives. It has therefore been proposed to utilize certain more readily soluble derivatives of these compounds in which at least one of the hydrogens in the acidic hydroxy groups has been replaced by a cation to form a water soluble salt.

In U.S. Patent 3,799,758 it was claimed that for NPMG acid only very specific salt forming cations, i.e. those wherein the "salt forming cation selected from the groups consisting of cations of alkali metals, alkaline earth metals, copper, zinc, manganese, nickel, ammonium and organic ammonium selected from primary, secondary and tertiary-alkyl, alkenyl

and alkynyl - amines, none of these having more than two amine groups; primary aryl amines, primary aryl diamines and heterocyclic amines and the acid addition salts of the above" were suitable for forming commercial phytotoxicant compositions.

This is also consistant with the possible salt forming groups defined in such related patents as U.S. Patents 3,455,675, 3,556,762 and 4,062,669 which concern salts of NPMIDA and related compounds and also with the salt forming groups mentioned in Eur. Pat. 48436 and German Patents 2,717,440 and 3,122.690 dealing with salts of phosphinothricine.

Included in the scope of said patents is N-Phosphonomethylglycine mono isopropyl amine (NPMG.IPA) which is marketed by the Monsanto company under the trade mark ROUNDUP(R) and which is recognized today as one of the leading herbicides in the world and ammonium phosphinothricine which is being developed by HOECHST company under the trade mark GLUPHOSINATE as a new contact herbicide.

Recently there have been discovered two additional novel groups of NPMG salts which are useful in phytotoxic compositions for controlling vegetation: the first group are N-Phosphonomethylglycine trialkyl sulfonium and trialkyl phosphonium salts (U.S. Patents 4,315,765 and 4,341,549) and the second group are NPMG iminourea, diiminourea, isothiourea and diisothiourea salts (Israel specifications 65187 and 66494).

As is noted from the above patents all the salt forming cations that have been used to produce water soluble salts of the above acids do not exhibit herbicidal or plant growth

regulating properties. Thus, the main phytotoxic properties of these salts result from theherbicidal activity of the aminoacid moiety.

It has now been surprisingly discovered that 3-amino-1,2,4-triazole (aminotriazole), which is a non-selective herbicide absorbed by roots and leaves, can serve as a salt forming cation for the above aminophosphonic and aminophosphinic acids thus resulting in novel per se double herbicidal action water soluble salts, which salts are very useful herbicides for controlling vegetation.

Thus according to the present invention there are now provided Aminotriazole derivatives of the general formula I

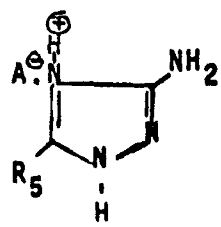

I

wherein $R_5$ is hydrogen or a branched or straight chain alkyl group having up to 6 carbon atoms and $A^-$ is selected from the group consisting of aminophosphonic acid anions, dianions and trianions and aminophosphinic acid anions and dianions of the formulas:

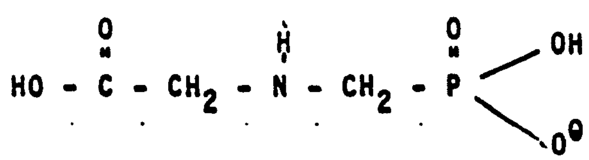

II

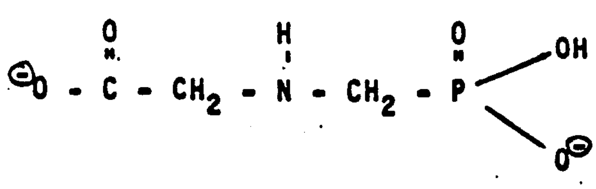

III

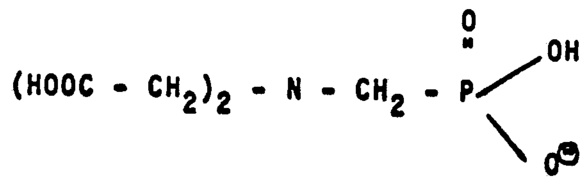

IV

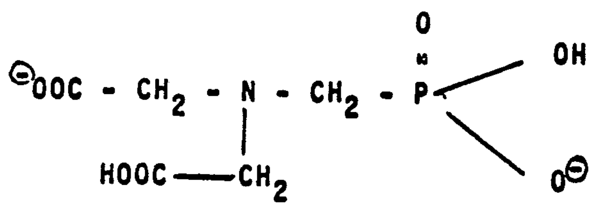

V

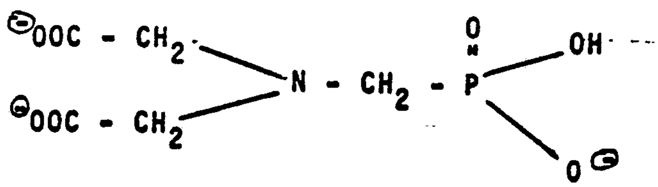

VI

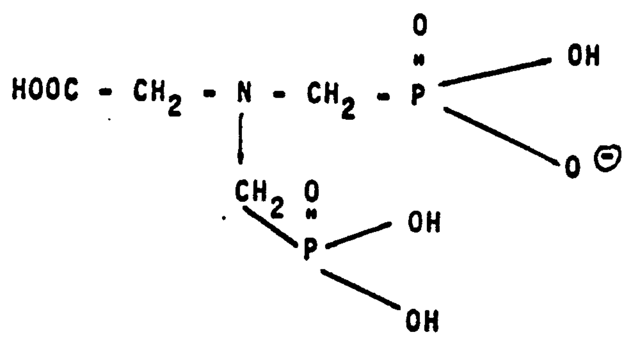

VII

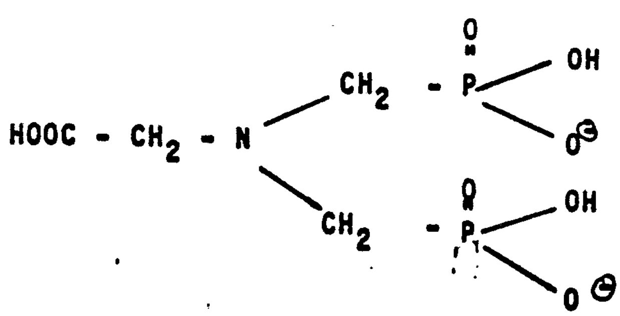

VIII

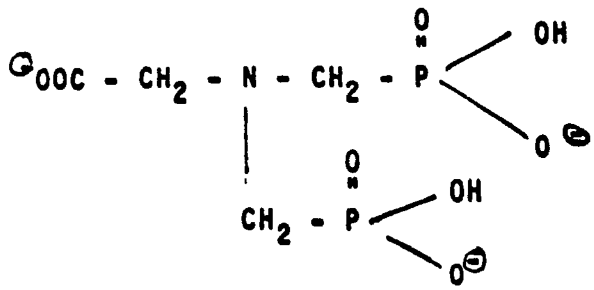

IX

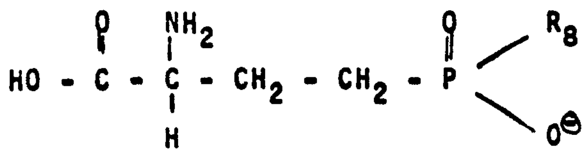

X

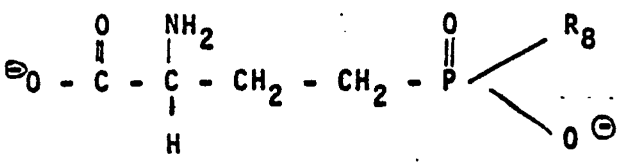

XI

and $R_8$ is an alkyl or halomethyl group.

Some preferred compounds according to the present invention are:

N-phosphonomethyliminodiacetic di(3-amino-1,2,4-triazole);

N-phosphonomethylglycine - di(3-amino-5-methyl-1,2,4-triazole);

N-N-bis (Phosphonomethyl)glycine mono (3-amino-1,2,4-triazole);

2-amino-4-(methylphosphino)-butanoic acid- di(3-amino-1,2,4-triazole); and

2-amino-4-(chloromethyl phosphino-butanoic acid- di(3-amino-1,2,4-triazole).

Another alternative way of referring to the novel salts of the present invention is to refer to formulas based on the aminophosphonic acid and aminophosphinic acid moiety.

Thus the present invention can alternatively be defined as referring to aminophosphonic acid derivatives of the general formula XII:

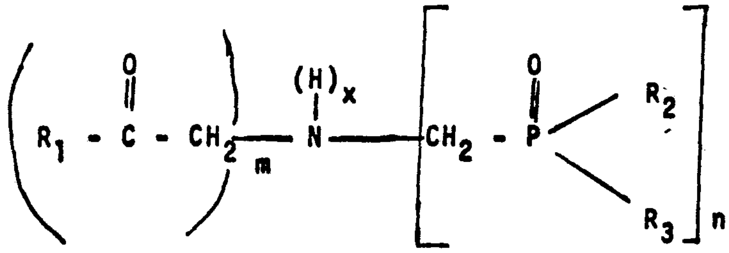

XII

wherein $R_1$, $R_2$ and $R_3$ are independently selected from OH and $OR_4$ at least one of $R_1$, $R_2$ or $R_3$ being $OR_4$ and/$R_4$ being a salt forming cation aminotriazole derivative of the general formula XIII:

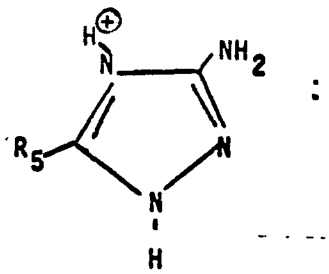

XIII

in which $R_5$ is H or straight or branched chain alkyl provided that:

a) X is zero or one.

b) When X is 0 m and n are each one or two provided that the sum of m and n is three and no more than three of $R_1$, $R_2$ and $R_3$ are $OR_4$.

c) When X = 1 m and n are each equal to 1 and no more than two of $R_1$, $R_2$ or $R_3$ are $OR_4$.

The aminophosphinic acid of the above invention are of the general formula XIV:

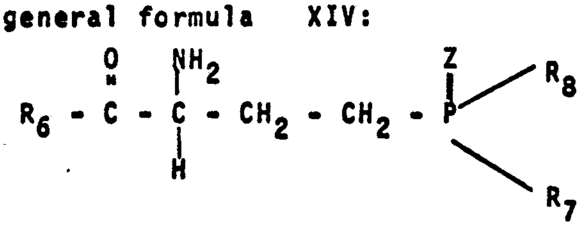

XIV

wherein $R_6$ and $R_7$ are independently selected from OH and $OR_4$, at least one of $R_6$ or $R_7$ being $OR_4$ and $R_4$ is a salt forming cation aminotriazole derivative of the general formula XIV as above defined, $R_8$ is alkyl or halomethyl, Z is O or S .

The above novel mono di and tri salts of aminotriazole derivatives are very water soluble and exhibit superior herbicidal activity when compared with the appropriate highly successful amine and ammonium salt. In comparing NPMG-Diaminotriazole salt of the present invention with NPMG.IPA salt (ROUNDUP$^{(R)}$) the following advantages of the above novel aminotriazole salt can be listed:

a) The NPMG-aminotriazole is an oil with higher solubility than the IPA salt (crystal in nature), thus while the solubility of NPMG.IPA is 480 g/l at room temperature and crystalization of the salt may occur at lower temperature (0-5$^{o}$c), the solubility of aminotriazole salt is higher (at least 1000 g/l) and no crystalization at 0$^{o}$c was observed. Accordingly, the foliage penetration and translocation of the aminotriazole salt in plants is superior because uptake is less efficient if the active material ingredient crystallizes or precipitates onto the leaf surface.

b) Due to the fact that in the NPMG-aminotriazole salt both the cation and anion exhibits phytotoxic properties, very high concentrations of active ingredient can be obtained in a small volume, i.e., in NPMG.IPA (ROUNDUP$^{(R)}$) each liter contains 480 g NPMG.IPA which in turn contains 360 g/l active ingredient while in the presently claimed aminotriazole salt 1000 g or more of the salt can be solubilized in a liter and the total amount introduced is of a phytotoxially active ingredient whereby about 1/3 liter according to the present invention contains as many grams of active ingredients as a whole liter containing the prior art compound.

c) The aminotriazole salt exhibits superior and faster foliage phytotoxic action during the first two weeks after application thus indicating that there is a heretofore unexpected and unreported synergistic effect between the cation and the anion in the aminotriazole-NPMG salt. (see example 4 hereinafter).

d) In controlling of some plant species lower amount of active material is needed when using aminotriazole salt compared to the IPA salt (the comparison was based on equal amount of NPMG acid equivalent per hectare).

e) NPMG-IPA salt shows little herbicidal activity when applied to the soil while the above NPMG-aminotriazole salts exhibit soil phytotoxic activity (see example 4).

In U.S. Patents 3,799,758 and 4,397,676 there appear statements with regard to the possibility of admixing the novel N-phosphonomethylglycine salts of said patents with other materials, e.g., fertilizers, other phytotoxicants, etc. which can be applied in a single application. In both of said patents 3-amino 1,2,4-triazole is mentioned inter alia as such a possible additive.

Thus, e.g., in column 14 of U.S. Patent 3,799,758 there appears the statement that "chemicals useful in combination with the active ingredients of this invention either simultaneously of sequentially include for example triazines, ureas, carbamates, acetamides, acetanilides, uracils, acetic acids, phenols, thiolcarbamates, triazoles, benzoic acids, nitriles and the like". This statement is followed by a list of 38 possible chemicals including 3-amino-1,2,4-triazole.

It is to be noted, however, that despite the above quoted suggestion of mixing found in the Patent literature, in the information pamphlet published by Monsanto Ltd. Agricultural Division, Thames Tower, Leicester, concerning ROUNDUP(R) there appears a specific warning and instruction that "Do not tank mix ROUNDUP(R) with other chemicals such as extra wetter, adjuvants, oils, antifoaming agents or residual herbicides except when directed by Monsanto Ltd. as a reduced level of weed control may result".

It is to be further noted that there is no indication anywhere in said Patents that an N-phosphonomethyl glycine salt and the phytotoxic chemical 3-amino-1,2,4-triazole were ever in fact used sequentially or simultaneously and in fact the only photoxicant from said list found to be used in combination with an NPMG salt is 2', 6',-diethyl-N-methoxymethyl-2-chloroacetanilide sold together with NPMG.IPA under the name BRONCO(R) as described in European publication 0070702.

It is further to be recognized that with the exception of the above emulsion in which the two ingredients are marketed together, a suggestion of combination is in fact recognized in the art as a suggestion of sequential application or tank mixing.

In actual use, tank-mixtures suffer from the disadvantage that such mixtures require that the farmer must purchase and store two separate herbicides until actual preparation of the tank-mixture, which is an inconvenience. The farmer is also required to measure out varing amounts of the two different herbicides allowing for the possibility of mixing errors.

In addition, all of the 38 chemicals listed, including 3-amino-1,2,4-triazole, when admixed with prior art salts under normal tank mix conditions do not undergo chemical reaction with the known glyphosate salts to produce the presently claimed new products. Moreover, an experiment carried out to attempt to react an aqueous solution of NPMG.IPA salt with 3-amino-1,2,4-triazole in more extreme condition (100$^{o}$c, several hours) resulted in unreacted starting material as shown in comparative Example 6 hereinafter demonstrating that the present invention of NPMG-aminotriazoles salts are neither taught nor suggested by the prior art in the above U.S. Patents, since the compounds of the present invention are not formed even by one who in disregard of the warnings of Monsanto in its pamphlets would choose a 3-amino-1,2,4-triazole derivative out of the 38 possibilities listed in the above-mentioned patent for combinations with a known NPMG salt such as ROUNDUP$^{(R)}$.

Furthermore, as indicated hereinbefore and exemplified hereinafter, the novel salts of the present invention in which the 3-amino-1,2,4-triazole derivative is itself the salt forming cation possess surprising and unexpected advantages over the known prior art salts.

In addition, since no-tillage farming usually requires a mixture or combination of a contact herbicide for quick burn-down of existing vegetation and use of one or more residual herbicides to provide season long weed control, NPMG-Diaminotriazole salts which possess both of the above herbicidal properties can be employed per se to replace mechanical tillage in the preparation of a seedbed in no tillage farming.

As indicated, there has been interest in combining Roundup$^{(R)}$ in tank mixes and split application with other herbicides. Usually, however, Roundup$^{(R)}$ was found to be no longer active when combined with other herbicides. Phillips (Proc. North Cent. Weed Control Cont. 30:115 (1975)) reported that Atrazine$^{(R)}$, Propachlor$^{(R)}$ and non herbicidal wettable powders decreased Roundup$^{(R)}$ phytotoxicity on sorghum. Selleck (Proc. North Weed Sci. Soc. 29.327 (1975)) found tank mixed Amitrole$^{(R)}$ and Simazine$^{(R)}$ reduced the activity of Roundup$^{(R)}$ on smooth brome, while Bromacil$^{(R)}$, Diuron$^{(R)}$ and Amitrole$^{(R)}$ reduced Roundup$^{(R)}$ toxicity to common milkweed.

Recently Selleck and Baird from Monsanto Company (Weed Sc. 29 (2) 185-90, 1981) had found that Linuron$^{(R)}$, Chlorbromuron$^{(R)}$ or Metribuzin$^{(R)}$, when added to Roundup$^{(R)}$ reduced the control of quackgrass (agropyron repens). Furthermore, quackgrass control was reduced when Amitrole$^{(R)}$ (3-amino-1,2,4-triazole) was mixed with Roundup$^{(R)}$, or when applied separately.

It is to be noted that the new salts of the present invention, e.g., the di(aminotriazole) NPMG salt showed no antagonistic interaction when applied to many species of weed and especially towards quackgrass (see example 4, tables II and III hereinafter). Moreover, the present aminotriazole-NPMG salts exhibits superior effect in comparison with the NPMG.IPA salt (Roundup$^{(R)}$).

Therefore, the present invention is neither taught nor suggested by the prior art and on the contrary, based on the above prior art publications, one skilled in the art would assume that the aminotriazole-NPMG salts of the present invention would at best behave as a mixture of aminotriazole and Roundup$^{(R)}$ and thus have reduced effectiveness when compared to Roundup$^{(R)}$ by itself.

Thus the present invention provides a whole new class of highly effective and needed herbicidal compositions which are neither taught nor suggested by the prior art despite the wide-felt need for such compounds and compositions and the extensive research carried out with regard to the above aminoacids and their derivatives.

The aminophosphonic acid salts of the above formula are those prepared from the following acids:
N-Phosphonomethylglycine, N-Phosphonomethyliminodiacetic acid, N-N-bis (phosphonomethyl) glycine and the like.

The aminophophinic acid salts of the above formula XIV are those prepared from the following acids:
2-amino-4-(methyl-phosphino)-butanoic acid, 2-amino-4-(chloro-methylphosphino)-butanoic acid, 2-amino-4-(ethyl-phosphino)-butanoic acid and the like.

The salt forming cation of the above formula XIII are those prepared from the following aminotriazole derivatives:
3-amino-1,2,4-triazole, 3-amino-5-methyl-1,2,4-triazole, 3-amino-5-ethyl-1,2,4-triazole and the like.

Preferred aminophosphonic-aminotriazole salts are those of the above formula XII wherein m=n=x=1, $R_3$ is OH and $R_1$ and $R_2$ are $OR_4$ and $R_4$ is a salt-forming cation of the above formula XIII in which $R_5$ is Hydrogen or alkyl; those of the formula XII wherein m=n=x=1, $R_1$ and $R_3$ are OH and $R_2$ is $OR_4$ and $R_4$ is as defined above; those of the above formula XII wherein m=2, n=1, x=o, R is OH and $R_1$ and $R_2$ are $OR_4$ and $R_4$ is as defined above; those of the above formula XII wherein m=2, n=1, x=o and $R_1$, $R_2$ and $R_3$ are $OR_4$ and $R_4$ is as defined above; and those of the above formula XII wherein m=1, n=2, x=o, $R_3$ is OH and $R_1$ and $R_2$ are $OR_4$ and $R_4$ is as defined above.

Especially preferred aminophosphonic acid salts are the following:
NPMG-Di (3-amino-1,2,4-triazole), NPMG-Mono-(3-amino-1,2,4-triazole),
N-Phosphonomethyl iminodiacetic acid-Di (3-amino-1,2,4-triazole), and
N-Phosphonomethyl iminodiacetic acid-tri (3-amino-1,2,4-triazole).

Preferred aminophosphinic-aminotriazole salts are those of the above formula XIV wherein $R_8$ is Methyl or Chloromethyl, Z is oxygen and $R_6$ and $R_7$ are $OR_4$ and $R_4$ is a salt forming cation of the above formula in which $R_5$ is Hydrogen or alkyl; and those of the above formula XIV wherein $R_8$ is Methyl or Chloromethyl, Z is oxygen, $R_6$ is Hydrogen and $R_7$ is $OR_4$ and $R_4$ is as defined above.

Especially preferred aminophosphinic acids salts are phosphinothricine -mono (3-amino-1,2,4-triazole) salt and phosphinothricine di (3-amino-1,2,4-triazole) salt.

The aminotriazole salts of the above aminoacid of the present invention can be prepared by forming an admixture of the free acid and the appropriate aminotriazole in water and heating the mixture until a clear solution is obtained.

For the preparation of a herbicidal solution, this clear solution, obtained as described above, can be used directly.

It is to be noted that although aminotriazole (a) is amphoteric it forms salts with the above aminoacids by undergoing protonation predominately on the imine nitrogen (N-4 of the ring) thus yielding a cation which is stabilized by amidinium type resonance (b).

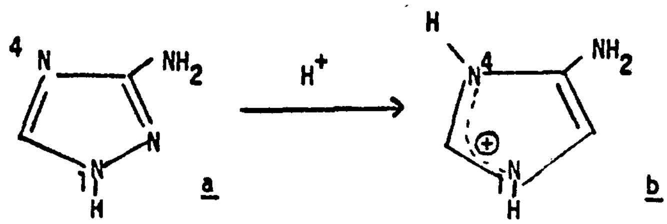


The resonance stabilization energy of the above cation serves as the driving force to promote the above reaction of aminotriazole and the aminoacids.

The present invention also relates to a new method of producing the above aminoacid-aminotriazoles salts. More particularly, this invention relates to the production of the above salt by condensation of the appropriate aminoguanidine salt of the above amino acid with low molecular weight carboxylic acid.

Thus in accordance with the above process the aminophosphonic acids of the above formula XII or the aminophosphinic acid of the above formula XIV are mixed in water with aminoguanidine bicarbonate and the mixture is heated to 70-80°C until a clear solution is obtained. The resulting amino guanidine salt is heated to 100-110°c and then the carboxylic acid (e.g. formic, acetic, propyonic etc.) is added, the solution is refluxed for 5-15 hours. The aminotriazole salt is then isolated by evaporation of the water.

It is believed that the process takes place in accordance with the following equations (taking NPMG as the amino acid);

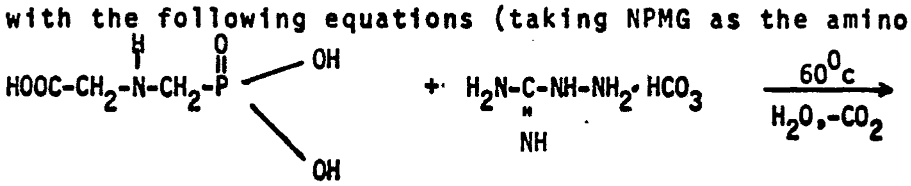

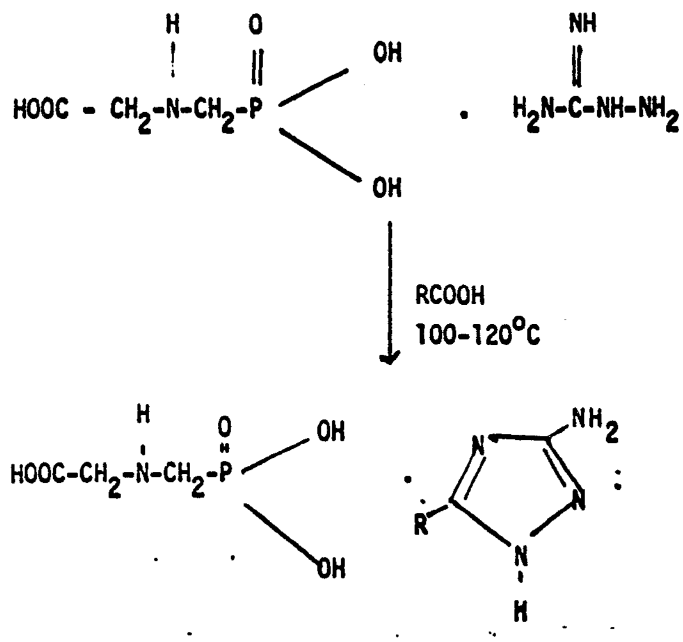


In conducting said process the temperature of the condensation can be from as low as 80 to 120°C or even higher, it is preferred to conduct said reaction at reflux temperature.

The time of reaction is not narrowly critical and can be from 5 hours heating time to as high as 24 hours. Of course it is obvious to those skilled in the art that the yield of the product will vary with the reaction time and the temperature of the reaction.

The process is carried out in an aqueous media, it is preferred to employ a saturated solution of the aminoacid aminoguanidine salt. However, the process is also operable at lower or higher concentration in water.

The ratio of reactants, that is the carboxylic acid and the aminoguanidine salt is not narrow, as it is apparent from the above equation, for best yields one should employ at least a stiochiometric amount of condensation agent; but in actual

practice, to obtain the best yield one employs 1 to 1.5 moles of carboxylic acid for each mole of aminoguanidine salt.

The carboxylic acids employed in said process are those having low molecular weight aliphatic carboxylic acid, i.e. having a molecular weight below 120 such as: Formic, acetic, propionic, isopropionic, butyric and the like.

Aminotriazole-NPMG salts can be also obtained by oxidation of the N-Phosphonomethyliminodiacetic acid-Aminotriazole salts with an oxidizing agent such as Hydrogen Peroxide or a free oxygen containing gas with catalyst such as activated carbon or metal catalyst.

In Israel Patent 42393 there is described and claimed a process for the production of N-Phosphonomethylglycine which comprises forming an admixture of N-(phosphonomethyl) iminodiacetic acid, water and an oxidizing agent and heating said admixture to a temperature at which said oxidizing agent and said N-(phosphonomethyl) iminodiacetic acid react to produce said N-phosphonomethylglycine.

Similarly in Israel Patent 47202 there is described and claimed a process for the production of N-phosphonomethylglycine which comprises contacting an aqueous solution of N-phosphonomethylimino diacetic acid with a molecular oxygen-containing gas at a temperature sufficiently elevated to initiate and sustain reaction and in the presence of a catalyst consisting essentially of activated carbon.

The novel amino triazole NPMG compounds of the present invention can be prepared in a manner similar to that described in said patents.

The compounds of the present invention can be used individually, or in an admixture of two or more compounds and are effective as post and pre emergent phytotoxicants or herbicides characterized by broad spectrum activity, i.e. they control the growth of a wide variety of plants including but not limited to ferns, conifer (pine, fir and the like) aquatic, monocotyledons and dicotyledons.

Thus a phytotoxic composition according to the present invention will comprise an adjuvent and an effective amount of one or more aminotriazole salts according to the present invention. Especially preferred are phytotoxic compositions wherein at least 50% of the active ingredient therein is a 3-amino-1,2,4-triazole salt of an aminophosphonic or aminophosphinic acid as defined herein.

While the invention will now be described in connection with certain preferred embodiments in the following examples it will be understood that it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the pactice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

In the following examples and throughout the specification parts and percents are by weight unless otherwise indicated.

EXAMPLE 1

General procedure for preparing aminotriazole salts of amino phosphonic and amino phosphinic acids.

A mixture of 0.2 mole of the aminoacid, 100 parts of water and 0.2-0.6 mole of the appropriate aminotriazole was agitated in a suitable reaction vessel at 50-60°C. After dissolution was complete as indicated by clarification of the reaction mixture the reaction mixture was concentrated on a steam bath at reduced pressure to yield a clear oil or white solid. The identity of the product was confirmed by Nuclear Magnetic Resonance (NMR) and Infrared spectrocopy.

Following the above procedure the following salts were prepared [NMR: $D_2O$ as solvent; δ, ppm, relative to TMS (tetramethylsilane)]

N-Phosphonomethylglycine di (3-amino-1,2,4-triazole)-oil, NMR: 7.82 (S,2H); 3.7 (S,2H): 3.18 (d,2H,J=13$H_z$).

N-Phosphonomethylglycine mono (3-amino-1,2,4-triazole)- white hygroscopic solid m.p. 127-129°c (dec.) NMR: 7.87 (S,1H); 3.64 (S,2H); 3.14 (d,2H,J=13$H_z$).

N-Phosphonomethylglycine di (3-amino-5-Methyl - 1,2,4-triazole) - oil NMR: 3.72 (S,2H); 3.15 (d,2H,J=13$H_z$); 1.96 (S,6H).

N-Phosphonomethylglycine mono (3-amino-5-ethyl-1,2,4-triazole) - white solid NMR: 3.6 (S,2H); 3.1 (d,2H, J=13$H_z$); 2.15 (q,2H, J=7$H_z$); 1.0 (t,3H,J=7$H_z$).

N-Phosphonomethyliminodiacetic di (3-amino-1,2,4-triazole) - oil
NMR: 7.83 (S,2H); 3.83 (S,4H); 3.3 (d,2H,J=12$H_z$)

N-Phosphonomethyliminodiacetic di (3-amino-5-methyl-1,2,4-triazole)-
NMR: 3.81 (S,4H); 3.3 (d,2H,J=12$H_z$); 1.94 (S,6H).

N-N-bis (phosphonomethyl) glycine mono (3-amino-1,2,4-triazole)-
crystalline hygroscopic solid, NMR: 7,78 (S,1H); 4.13 (S,2H);
3.5 (d,4H,J=12$H_z$).

N-N-bis (phosphonomethyl)glycine di (3-amino-1,2,4-triazole) - oil
NMR: 7.92 (d,2H); 3.92 (S,2H); 3.52 (d,4H,J=12$H_z$).

2-amino-4-(methyl phosphino)-butanoic acid mono (3-amino-1,2,4-triazole).
7,9 (S,1H); 3.85 (1H,t,J=6$H_z$); 1.7-2,4 (m,4H); 1.40 (t,3H,J=15$H_z$).

2-amino-4-(methyl phosphino)-butanoic acid di (3-amino-1,2,4-triazole)
7.85 (S,2H); 3.76 (1H, t, J=6$H_z$); 1.6-2,4 (m,4H); 1.37 (t,3H,J=15$H_z$).

EXAMPLE 2

A series of runs were made to prepare aminoacid-aminotriazole salts by the condensation of the Aminoguanidine salt with carboxylic acid.

A mixture of 0.2 mole of the amino acid, 90 parts of water and 0.2-0.4 mole of amino guanidine bicarbonate was agitated in a suitable reaction vessel at 60°c. After dissolution was complete, as indicated by clarification of the reaction mixture and the ceasing of evolution of carbon dioxide, the solution was heated to 100-110°c, 0.2-0.5 mole of the appropriate carboxylic acid was added and the solution was refluxed for 5-15 hours. The solution was concentrated on a steam bath at reduced pressure to yield a clear oil or white solid which was analyzed by NMR and IR spectral analysis. Table I gives the result of these experiments.

TABLE I.

| EXPERIMENT NO. | CARBOXYLIC ACID (mole x $10^{-1}$) | AMINOGUANIDINE (mole x $10^{-1}$) | AMINOACID (0.2 mole) | REACTION TIME (h) | MOLE % AMITRIAZOLE SALT. |
|---|---|---|---|---|---|
| 1 | FORMIC (4.2) | 4 | NPMG | 5 | 80 |
| 2 | FORMIC (2.2) | 2 | -"- | 3 | 82 |
| 3 | FORMIC (4.4) | 4 | NPMIDA | 6 | 90 |
| 4 | FORMIC (4.0) | 4 | PHOSPHINOTHRICINE | 9 | 70 |
| 5 | ACETIC (4.8) | 4 | NPMIDA | 8 | 80 |
| 6 | ACETIC (2.1) | 2 | NPMG | 6 | 75 |
| 7 | ACETIC (4.2) | 4 | NPMG | 10 | 55 |
| 8 | ACETIC (4.2) | 4 | PHOSPHINOTHRICINE | 6 | 65 |
| 9 | PROPIONIC(2.3) | 2 | NPMG | 8 | 35 |
| 10 | PROPIONIC(4.4) | 4 | NPMIDA | 10 | 70 |

EXAMPLE 3

Using a low pressure apparatus consisting of a pair of shakers to provide agitation 0.5 g of catalyst (Norit A or 5% Pt/C), 0.02 mole N-Phosphonomethyl imino diacetic - DI (3-amino-1,2,4-triazole) salt and 50 g distilled water were charged into the bottle and heated to 100°C-105. The bottle was sealed and depressurized several times with $O_2$ at 2.3 kg/cm$^2$ to remove the air. The reaction was conducted at 2.3 kg/cm$^2$ for 2.5 hours. After the reaction was terminated, the catalyst was filtered off and the filtrate evaporated under reduced pressure to yield an oil product. The oil product was analysed by NMR and was found to be identical to that produced in Example 1. Following the above procedure other salts of NPMG can be prepared, e.g., N-Phosphonomethylglycine-mono (3-amino-1,2,4-triazole), N-Phosphonomethylglycine mono (3-amino-5-methyl-1,2,4-triazole), N-Phosphonomethylglycine Di(3-amino-5-methyl-1,2,4-triazole) and the like.

EXAMPLE 4

The post-emergence herbicidal activity of various compounds of the present invention is demonstrated as follows: The active ingredients are applied in spray to 21 day old specimens of various plant species. The spray, a water solution containing active ingredient and a surfactant is applied to the plants in different sets of pans at several rates (kilogram per dunam) of active ingredient. The treated plants are placed in a greenhouse and the effects are observed and recorded after 4 days (table II) and after 2 weeks (table III). The post-emergence herbicidal activity index used in Table II and III is as follows:

| PLANT RESPONSE | INDEX | PLANT RESPONSE | INDEX |
|---|---|---|---|
| No injury | 0 | Severe injury | 3 |
| Slight injury | 1 | Killed | 4 |
| Moderate injury | 2 | | |

The plant species utilized in these tests are identified by letter in accordance with the following legend:

A. Polygonum equiseliforme

B. Rubus Sanguineus

C. Inula Viscosa

D. Phragmites australis

E. Pennisetum clandestinum

F. Cynodon daotylon

G. Convolvulus arvensis

H. Avena sterilis

I. Cyperus Rotundus

J. Agropyron repens (quackgrass)

| | TABLE II | | | | | | | | | | TABLE III | | | | | | | | | |
|---|---|---|---|---|---|---|---|---|---|---|---|---|---|---|---|---|---|---|---|---|
| Compound | A | B | C | D | E | F | G | H | I | J | A | B | C | D | E | F | G | H | I | J |
| I | 0 | 1 | 1 | 1 | 2 | 1 | 1 | 2 | 2 | 1 | 3 | 3 | 3 | 3 | 3 | 3 | 3 | 4 | 4 | 4 |
| II | - | - | - | - | - | - | - | - | - | - | 3 | 2 | 3 | 3 | 3 | 2 | 2 | 3 | 3 | 2 |
| III | - | - | - | - | - | - | - | - | - | - | 3 | 3 | 3 | 2 | 3 | 3 | 3 | 3 | 3 | 3 |
| IV | 0 | 0 | 0 | 0 | 1 | 1 | 0 | 1 | 1 | 1 | 3 | 2 | 2 | 2 | 3 | 3 | 3 | 3 | 3 | 3 |
| V | - | - | - | - | - | - | - | - | - | - | 1 | 1 | 2 | 1 | 1 | 0 | 0 | 1 | 1 | 1 |

The following are the compounds referred to by Roman numerals in the table:

| ROMAN NUMERAL | COMPOUND NAME | ACTIVE INGREDIENT mole/l. |
|---|---|---|
| I | Di(3-amino-1,2,4-triazole)NPMG salt | 0.022 |
| II | Di(3-amino-1,2,4-triazole)NPMIDA salt | 0.043 |
| III | Di(3-amino-1,2,4-triazole)Phosphinothricine salt. | 0.03 |
| IV | Mono(Isopropylamine)NPMG salt (ROUNDUP$^{(R)}$) | 0.043 |
| V | Mono(Isopropylamine)NPMIDA salt | 0.043 |

It is to be noted from the above results that the aminotriazole salts of the above aminoacid are more active that the related amine salt i.e. I with half mole amount of active material exhibits at least the same result as one mole of IPA salt IV and II exhibit far more superior activity than the IPA salt IV.

Moreover, the aminotriazole salt I exhibit faster phytotoxic action (knock down effect) when comparing the appropriate amine salt (IV) in the first 4 days after application (table II).

EXAMPLE 5

Plastic trays were planted with green cynondon dactylon, convolvulus arvensis and sinapis arvensis and covered with a good grade of top soil. All materials were applied with a laboratory sprayer that delivered a volume of 187 l/hectare. The trays of soil were thereafter watered lightly to activate the aminotriazole salts. The experiment was conducted twice and each was arranged in a completely random design with three replications. Visual estimates of percent inhibition were made at two weeks after treatment. The results are summarized in table IV.

TABLE IV

| ACTIVE INGREDIENT | RATE KG/HECTARE | % inhibition. A* | B | C |
|---|---|---|---|---|
| I | 0.5 | 45 | 80 | 60 |
| | 1.0 | 80 | 90 | 90 |
| II | 0.7 | 40 | 55 | 65 |
| | 1.2 | 70 | 70 | 80 |
| III | 1 | 10 | 30 | 30 |
| | 1.5 | 20 | 50 | 60 |

*A - cynodon dactylon
B - convolvulus arvensis
C - sinapis arvensis.

COMPARATIVE EXAMPLE 6

A) About 17 parts (0.1 mole) of N-Phosphonomethylglycine were added to a solution of 6 parts (0.1 mole) of Isopropylamine dissolved in 50 parts of water and contained in a suitable reaction vessel. The reaction mixture cleared within a short period of time while the mixture was subject to agitation. The resulting solution was then concentrated by heating to 90°c at reduce pressure. The residue was a viscous oil which was identified as the Mono-Isopropylamine salt of NPMG by NMR spectroscopy. NMR ($D_2O$, $\delta$, ppm, relative to TMS): 3.59 (S,2H); 3.35 (heptate, 1H, $J=6H_z$); 3.05 (d, 2H, $J=12H_z$); 1.12 (d,6H,$J=6H_z$).

B) About 2.3 g (0.01 mole) of Mono-Isopropylamine salt from A were added to a solution of 1.8 part (0.02 mole) of 3-amino-1,2,4-triazole dissolved in 5 parts of water contained in a suitable reaction vessel, the reaction mixture cleared within a short period of time and the solution was heated to 100°c for 2 hours. The resulting solution was then concentrated by heating to 90°c at reduce pressure.

The residue was a viscous oil which was confirmed as the two starting materials (NPMG-IPA and aminotriazole) by NMR spectroscopy: NMR ($D_2O$, δ, ppm, relative to TMS): 3.6 (S,2H); 3.33 (heptate, 1H, J=6$H_z$); 3.03 (d,2H,J=12$H_z$) and 1.14 (d, 6H, J=6$H_z$) for mono-isopropylamine salt of NPMG and 7.63 (S,2H) for 3-amino-1,2,4-aminotriazole.

The compositions of this invention provide a wide spectrum of weed control and are also extremely useful as general herbicides as well as in controlling unwanted plants in orchards, tree farms and various crops. For example, it has been found that by directing a spray of the compositions of this invention at the unwanted plants while essentially preventing such spray from contacting the leaves of trees, that such unwanted plants are controlled while there is no apparent injury to the trees.

The compositions of this invention are also useful for control of weeds between cropping seasons, for the renovation of stale seed beds and the like.

In applying the compositions of this invention to the plants which it is desired to control, it has been found to be desirable that the plant be emerged from the ground and even more desirable that the plant be at least at the 2 leaf stage for maximum effect.

It has been found that when the plants to be controlled have a portion of their growth above the ground or water, and the above-ground or above-water portion of the plant is contacted with the herbicidal compositions of this invention at appropriate rates, the herbicide is translocated to kill such plant parts which are below the ground or water surface.

One can obtain limited selectivity in crops by directing the spraying of a composition of this invention at a selected concentration on vegetation around the base of plants with minimal spray contact with the leafy portions of the crop plants. The directed spraying can be done with or without a protective device to prevent contact of the spray with the leaves of crop plants.

A non-exhaustive list of some of the plant species which are controlled by the compositions of this invention, in addition to those shown in Table I, are set forth below:

CRAMBE HISPANICA
ERUCARIA MYAGROIDES
HIRCHFELDIA INCANA
DIPLOTAXIS ERUCOIDES
AMARANTHUS RETROFLEXUS L
" LIVIDUS L
" ALBUS L
" GRAECIZANS
ECHINOCHLOA COLONUM
CYNODON DACTYLON
PHALARIS
LOLIUM RIGIDUM
CHENOPODIUM MURALE
BETA VULGARIS
CHANOPODIUM OPULIFOLIUM
SONCHUS OLERACEUS
XANTHIUM STRUMARIUM
CALENDULA ARVENSIS
RIDULFIA SEGETUM
TORDYLIUM AEGYPTIACUM
BUPLEVRUM PERFOLIATUM
TRIGONELLA
VICIA VULGARE
EUPHORIA CYBIRENSIS
CROZOPHORA TINCTORIA
ERODIUM MOSCHATUM
RANUNCULUS TRACHYCARFUS
" ARVENSIS
SOLANUM ELEAGNIFOLIUM
POLYGONUM EQUISELIFORME
ANTHEMIS PSEUDOCOTULA
" MELANOLEPIS
CARTHAMUS TENUIS
ORMENIS MIXTA
EROGERON CRISPUS
SCORPIURUS MURICATA
HYMENOCARPUS CIRCINNATUS
SECURIGERA SECURIDACA
DIGITARIA SANGUINALIS
SORGHUM HALEPENSE
SETARIA VERTICILLATA
AVENA STERILIS
AMMI MAJOR
CONVOLVULVUS ARVENSIS

The phytotoxicant compositions, including concentrates which require dilution prior to application to the plants, of this invention contain at least one active ingredient and an adjuvant in liquid or solid form. The compositions are prepared by admixing the active ingredient with an adjuvant including diluents, extenders, carriers and conditioning agents known per se to provide compositions in the form of finely-divided particulate solids, pellets, solutions, dispersions or emulsions. Thus the active ingredient can be used with an adjuvant such as a finely-divided solid, a liquid of organic origin, water, a wetting agent, a dispersing agent, an emulsifying agent or any suitable combination of these.

From the viewpoint of economy and convenience, water is the preferred diluent, particularly with the highly watersoluble N-Phosphonomethylglycine and Phosphinothricine aminotriazole salts of the present invention. With these derivatives, solutions containing as high as 90% by weight of active materials can be readily prepared.

The phytotoxicant compositions of this invention, particularly liquids and soluble powders, preferably contain as a conditioning agent one or more surface-active agents in amounts sufficient to render a given composition readily dispersible in water or in oil. The incorporation of a surface-active agent into the compositions greatly enhances their efficacy. By the term "Surface-active agent" it is understood that wetting agents, dispersing agents, suspending agents and emulsifying agents are included therein. Anionic, cationic and non-ionic agents can be used with equal facility.

Preferred wetting agents are alkyl benzene sulfated fatty alcohols, amines or acid amides, sulfated or sulfonated fatty acid esters petroleum sulfonates, sulfonated vegetable oils, polyoxyethylene derivatives of alkylphonols (particularly isooctylphenol and nonylphenol), mono-fatty di-alkyl amine oxide, and mono-fatty di-alkyl benzalkonium chloride.

Preferred dispersants are methyl cellulose, polyvinyl alcohol, sodium lignin sulfonates, polymeric alkyl naphthalene sulfonates and sodium naphthalene sulfonate.

Water-dispersible powder compositions can be made containing one or more active ingredients, an inert solid extender and one or more wetting and dispersing agents. The inert solid extenders are usually of mineral origin such as the natural clays, diatomaceous earth and synthetic minerals derived from silica and the like. Examples of such extenders include kaolinites, and synthetic magnesium silicate. The water-dispersible compositions of this invention usually contain from about 10 to about 90 parts by weight of active ingredient, from about 0.5 to 20 parts by weight of wetting agent, from about 0.5 to 20 parts by weight of dispersant and from 5.0 to about 90 parts by weight of inert, solid extender, all parts being by weight of the total composition.

Aqueous suspensions can be prepared by mixing together and grinding an aqueous slurry of water-insoluble active ingredient in the presence of dispersing agents to obtain a concentrated slurry of very finely-divided particles. The resulting concentrated aqueous suspension is characterized by its extremely small particle size, so that when diluted and sprayed, coverage is very uniform.

Emulsifiable oils are usually solutions of active ingredient in water-immiscible or partially water-immiscible solvents together with a surface active agent. Suitable solvents for the active ingredient of this invention include hydrocarbons and water-immiscible ethers, esters or ketones. The emulsifiable oil compositions generally contain from about 10 to 95 parts active ingredient, about 2 to 50 parts surface active agent and about 4 to 90 parts solvent, all parts being by weight based on the total weight of emulsifiable oil.

The application of an effective amount of the compounds of this invention to the plant is essential and critical for the practice of the present invention. The exact amount of active ingredient to be employed is dependent upon the response desired in the plant as well as such other factors as the plant species, the stage of development thereof, and the amount of rainfall as well as the specific glycine employed. In foliar treatment for the control of vegetative growth, the active ingredients are applied in amounts from about 0.1 to about 50 or more kilogram per dunam. In applications for the control of aquatic plants, the active ingredients are applied in amounts of from about 0.1 parts per million to about 2000 parts per million, based on the aquatic medium. An effective amount for phytotoxicant or herbicidal control is that amount necessary for overall or selective control, i.e., a phytotoxic or herbicidal amount. It is believed that one skilled in the art can readily determine from the teachings of this specification, including examples, the approximate application rate.

It will be evident to those skilled in the art that the invention is not limited to the details of the foregoing illustrative embodiments and examples and that the present invention may be embodied in other specific forms without departing from the essential attributes thereof, and it is, therefore, desired that the present embodiments and examples be considered in all respects as illustrative and not restrictive, reference being made to the appended claims, rather than tothe foregoing description, and all changes which come with the meaning and range of equivalency of the claims are, therefore, intended to be embraced therein.

WHAT IS CLAIMED IS:

1. Aminotriazole derivatives of the general formula I

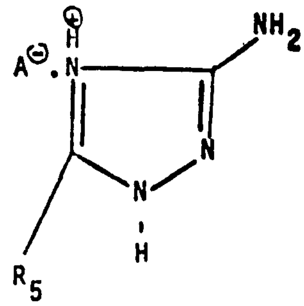

I

wherein $R_5$ is hydrogen or a branched or straight chain alkyl group having up to 6 carbon atoms and $A^-$ is selected from the group consisting of aminophosphoric acid anions, dianions and trianions and aminophosphinic acid anions and dianions of the formulas:

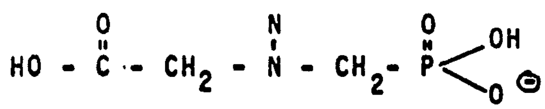

II

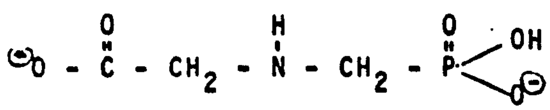

III

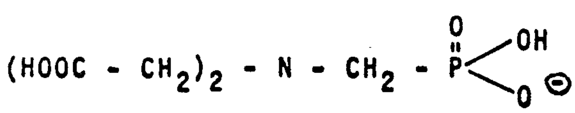

IV

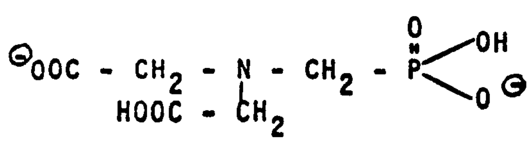

V

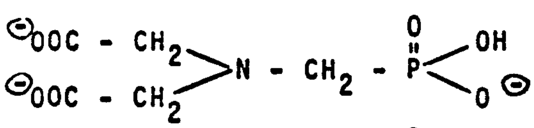

VI

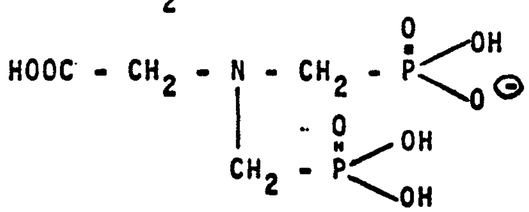

VII

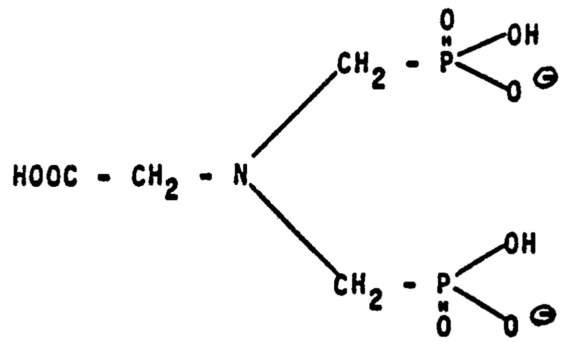

VIII

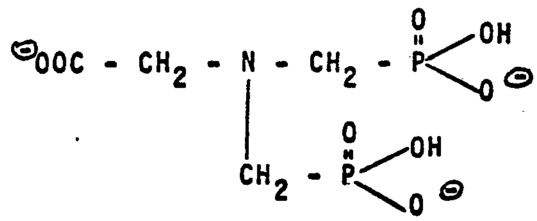

IX

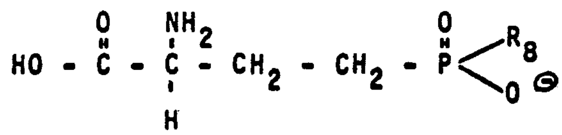

X

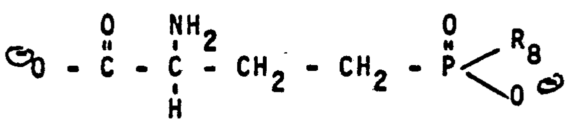

XI

and $R_8$ is an alkyl or halomethyl group.

2. N - phosphonomethylglycine-di-(3-amino-1,2,4-triazole).

3. N - phosphonomethylglycine-mono-(3-amino-1,2,4-triazole).

4. N-phosphonomethyliminodiacetic di-(3-amino-1,2,4-triazole).

5. N-phosphonomethylglycine-di-(3-amino-5-methyl-1,2,4-triazole).

6. N-N-bis (phosphonomethyl glycine mono-(3-amino-1,2,4-triazole).

7. 2-amino-4-(methylphosphino)-butanoic acid-di-(3-amino-1,2,4-triazole).

8. 2-amino-4-(chloromethyl phosphino)-butanoic acid-di-(3-amino-1,2,4-triazole).

9. A phytotoxic composition comprising an adjuvant and an effective amount of one or more aminotriazole derivatives as claimed in claim 1.

10. A phytotoxic composition wherein at least 50% of the active ingredient therein is a 3-amino-1,2,4-triazole salt of an aminophosphonic or aminophosphinic acid as claimed in claim 1.

11. A method of controlling vegetation comprising applying a phytotoxic amount of a compound as claimed in claim 1.

12. A process for preparing novel aminotriazole derivatives of the general formula I

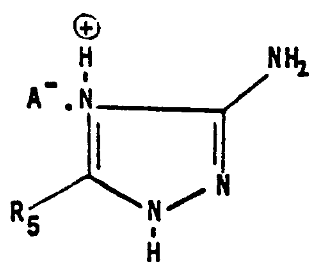

I

wherein $R_5$ is hydrogen or a branched or straight chain alkyl group having up to 6 carbon atoms and $A^-$ is selected from

the group consisting of aminophosphoric acid anions, dianions and trianions and aminophosphinic acid anions and dianions of the formulas:

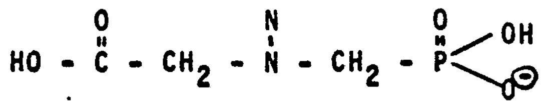

II

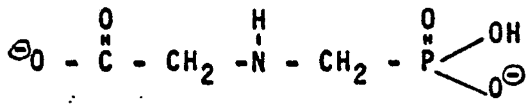

III

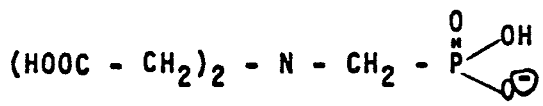

IV

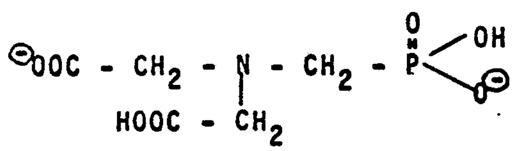

V

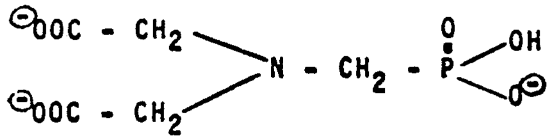

VI

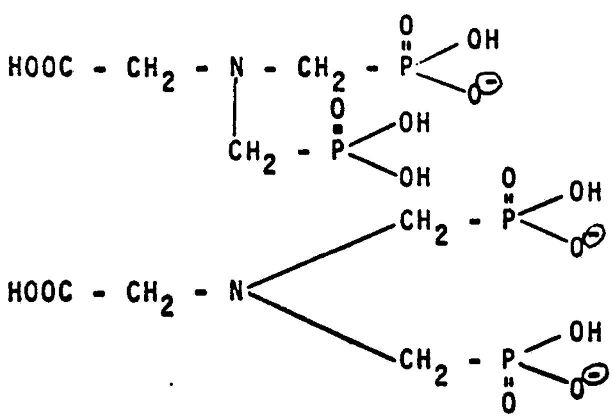

VII

VIII

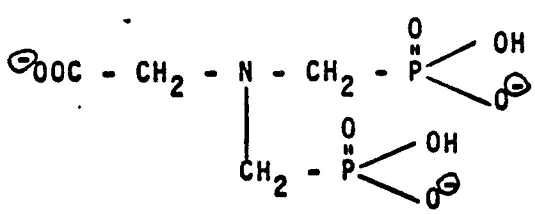

IX

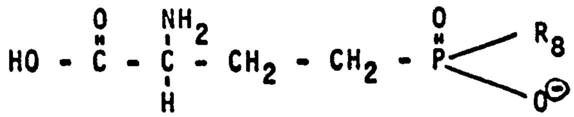

X

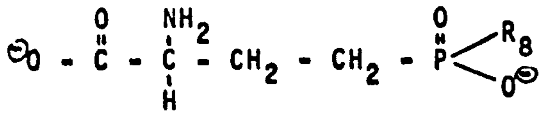

XI

and $R_8$ is an alkyl or halomethyl group, said process comprising:

a) reacting an aminophosphonic acid of the general formula XV

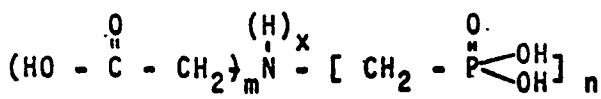

XV

in which

X is zero or one and

when X is o m and n are each one or two provided that the sum of m and n is three and

when x = 1 m and n are each equal to 1

or an

aminophosphinic acid of the general formula XVI

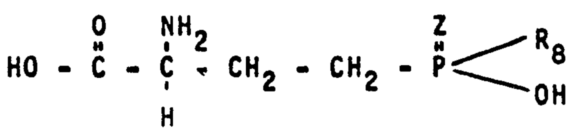

XVI

wherein Z is O or S and $R_8$ is alkyl or halomethyl, with an amino triazole derivative of the general formula XVII

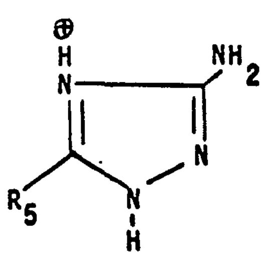

XVII

in which $R_5$ is H or straight or branched chain akyl, in aqueous solution; or

b) reacting an aminophosphinic acid of the general formula XV or an aminophosphinic acid of the general formula XVI with aminoguanidine bicarbonate to form the aminoguanidine salts of said acids and then reacting said formed salt with a low molecular weight carboxylic acid in aqueous solution at a temperature of about 80° to about 120°C; or

for preparing an aminotriazole derivative of the general formula I wherein $A^-$ is an aminophosphoric acid anion or dianion of the formula II or III comprising

c) oxidizing the corresponding N-phosphonomethyl-aminodiacetic acid aminotriazole salt with hydrogen peroxide or a free oxygen containing gas in the presence of a catalyst.

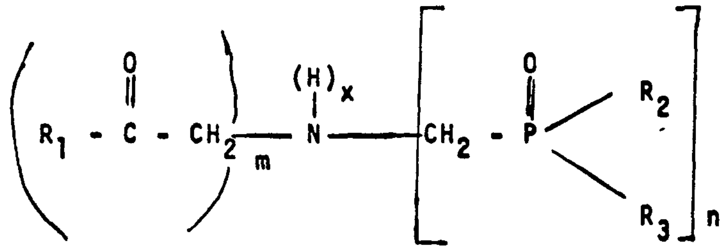

XII

wherein $R_1$, $R_2$ and $R_3$ are independently selected from OH and $OR_4$ at least one of $R_1$, $R_2$ or $R_3$ being $OR_4$ and $R_4$ being a salt forming cation aminotriazole derivative of the general formula XIII:

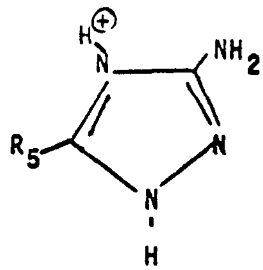

XIII

in which $R_5$ is H or straight or branched chain alkyl provided that:

a) X is zero or one.

b) When X is 0 m and n are each one or two provided that the sum of m and n is three and no more than three of $R_1$, $R_2$ and $R_3$ are $OR_4$.

c) When X = 1 m and n are each equal to 1 and no more than two of $R_1$, $R_2$ or $R_3$ are $OR_4$.

The aminophosphinic acid of the above invention are of the general formula XIV:

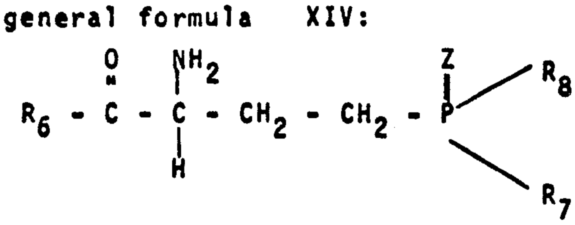

XIV

wherein $R_6$ and $R_7$ are independently selected from OH and $OR_4$, at least one of $R_6$ or $R_7$ being $OR_4$ and $R_4$ is a salt forming cation aminotriazole derivative of the general formula XIII as above defined, $R_8$ is alkyl or halomethyl, Z is O or S .

In addition, all of the 38 chemicals listed, including 3-amino-1,2,4-triazole, when admixed with prior art salts under normal tank mix conditions do not undergo chemical reaction with the known NPMG salts to produce the presently claimed new products. Moreover, an experiment carried out to attempt to react an aqueous solution of NPMG.IPA salt with 3-amino-1,2,4-triazole in more extreme condition (100$^{o}$c, several hours) resulted in unreacted starting material as shown in comparative Example 6 hereinafter demonstrating that the present invention of NPMG-aminotriazoles salts are neither taught nor suggested by the prior art in the above U.S. Patents, since the compounds of the present invention are not formed even by one who in disregard of the warnings of Monsanto in its pamphlets would choose a 3-amino-1,2,4-triazole derivative out of the 38 possibilities listed in the above-mentioned patent for combinations with a known NPMG salt such as ROUNDUP(R).

Furthermore, as indicated hereinbefore and exemplified hereinafter, the novel salts of the present invention in which the 3-amino-1,2,4-triazole derivative is itself the salt forming cation possess surprising and unexpected advantages over the known prior art salts.

In addition, since no-tillage farming usually requires a mixture or combination of a contact herbicide for quick burn-down of existing vegetation and use of one or more residual herbicides to provide season long weed control, NPMG-Diaminotriazole salts which possess both of the above herbicidal properties can be employed per se to replace mechanical tillage in the preparation of a seedbed in no tillage farming.

| PLANT RESPONSE | INDEX | PLANT RESPONSE | INDEX |
|---|---|---|---|
| No injury | 0 | Severe injury | 3 |
| Slight injury | 1 | Killed | 4 |
| Moderate injury | 2 | | |

The plant species utilized in these tests are identified by letter in accordance with the following legend:

A. Polygonum equiseliforme
B. Rubus Sanguineus
C. Inula Viscosa
D. Phragmites australis
E. Pennisetum clandestinum
F. Cynodon dactylon
G. Convolvulus arvensis
H. Avena sterilis
I. Cyperus Rotundus
J. Agropyron repens (quackgrass)

| | TABLE II | | | | | | | | | | TABLE III | | | | | | | | | |
|---|---|---|---|---|---|---|---|---|---|---|---|---|---|---|---|---|---|---|---|---|
| Compound | A | B | C | D | E | F | G | H | I | J | A | B | C | D | E | F | G | H | I | J |
| I | 0 | 1 | 1 | 1 | 2 | 1 | 1 | 2 | 2 | 1 | 3 | 3 | 3 | 3 | 3 | 3 | 3 | 4 | 4 | 4 |
| II | - | - | - | - | - | - | - | - | - | - | 3 | 2 | 3 | 3 | 3 | 2 | 2 | 3 | 3 | 2 |
| III | - | - | - | - | - | - | - | - | - | - | 3 | 3 | 3 | 2 | 3 | 3 | 3 | 3 | 3 | 3 |
| IV | 0 | 0 | 0 | 0 | 1 | 1 | 0 | 1 | 1 | 1 | 3 | 2 | 2 | 2 | 3 | 3 | 3 | 3 | 3 | 3 |
| V | - | - | - | - | - | - | - | - | - | - | 1 | 1 | 2 | 1 | 1 | 0 | 0 | 1 | 1 | 1 |

The following are the compounds referred to by Roman numerals in the table:

| ROMAN NUMERAL | COMPOUND NAME | ACTIVE INGREDIENT mole/l. |
|---|---|---|
| I | Di(3-amino-1,2,4-triazole)NPMG salt | 0.022 |
| II | Di(3-amino-1,2,4-triazole)NPMIDA salt | 0.043 |
| III | Di(3-amino-1,2,4-triazole)Phosphinothricine salt. | 0.03 |
| IV | Mono(Isopropylamine)NPMG salt (ROUNDUP(R)) | 0.043 |
| V | Mono(Isopropylamine)NPMIDA salt | 0.043 |

It is to be noted from the above results that the aminotriazole salts of the above aminoacid are more active than the related amine salt i.e. I with half mole amount of active material exhibits at least the same result as one mole of IPA salt IV and II exhibit far more superior activity than the IPA salt V.

Moreover, the aminotriazole salt I exhibit faster phytotoxic action (knock down effect) when comparing the appropriate amine salt (IV) in the first 4 days after application (table II).

EXAMPLE 5

Plastic trays were planted with green cynondon dactylon, convolvulus arvensis and sinapis arvensis and covered with a good grade of top soil. All materials were applied with a laboratory sprayer that delivered a volume of 187 l/hectare. The trays of soil were thereafter watered lightly to activate the aminotriazole salts. The experiment was conducted twice and each was arranged in a completely random design with three replications. Visual estimates of percent inhibition were made at two weeks after treatment. The results are summarized in table IV.

TABLE IV

| ACTIVE INGREDIENT | RATE KG/HECTARE | % inhibition. A* | B | C |
|---|---|---|---|---|
| I | 0.5 | 45 | 80 | 60 |
| | 1.0 | 80 | 90 | 90 |
| II | 0.7 | 40 | 55 | 65 |
| | 1.2 | 70 | 70 | 80 |
| III | 1 | 10 | 30 | 30 |
| | 1.5 | 20 | 50 | 60 |

*A - cynodon dactylon
B - convolvulus arvensis
C - sinapis arvensis.

COMPARATIVE EXAMPLE 6

A) About 17 parts (0.1 mole) of N-Phosphonomethylglycine were added to a solution of 6 parts (0.1 mole) of Isopropylamine dissolved in 50 parts of water and contained in a suitable reaction vessel. The reaction mixture cleared within a short period of time while the mixture was subject to agitation. The resulting solution was then concentrated by heating to 90$^o$c at reduce pressure. The residue was a viscous oil which was identified as the Mono-Isopropylamine salt of NPMG by NMR spectroscopy.
NMR ($D_2O$, $\delta$, ppm, relative to TMS): 3.59 (S,2H); 3.35 (heptate, 1H, J=6$H_z$); 3.05 (d, 2H, J=12$H_z$); 1.12 (d,6H,J=6$H_z$).

B) About 2.3 g (0.01 mole) of Mono-Isopropylamine salt from A were added to a solution of 1.8 g (0.02 mole) of 3-amino-1,2,4-triazole dissolved in 5 parts of water contained in a suitable reaction vessel, the reaction mixture cleared within a short period of time and the solution was heated to 100$^o$c for 2 hours. The resulting solution was then concentrated by heating to 90$^o$c at reduce pressure.

WHAT IS CLAIMED IS:

1. Aminotriazole derivatives of the general formula I

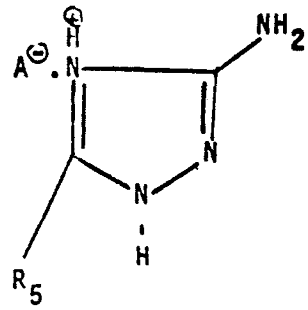

I

wherein $R_5$ is hydrogen or a branched or straight chain alkyl group having up to 6 carbon atoms and $A^-$ is selected from the group consisting of aminophosphonic acid anions, dianions and trianions and aminophosphinic acid anions and dianions of the formulas:

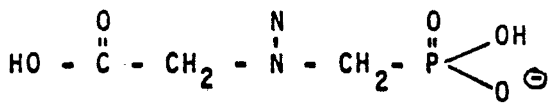

II

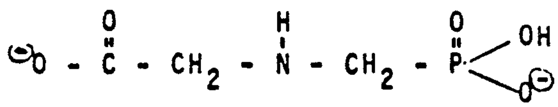

III

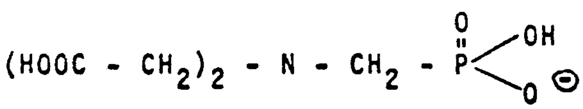

IV

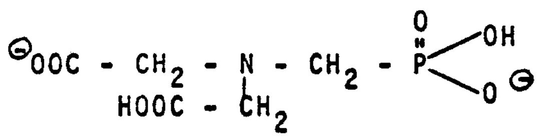

V

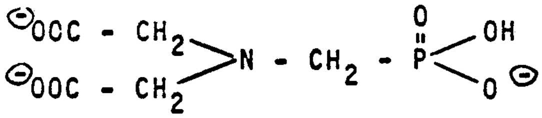

VI

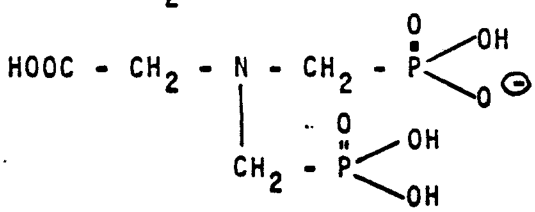

VII

in which $R_5$ is H or straight or branched chain akyl, in aqueous solution; or

b) reacting an aminophosphonic acid of the general formula XV or an aminophosphinic acid of the general formula XVI with aminoguanidine bicarbonate to form the aminoguanidine salts of said acids and then reacting said formed salt with a low molecular weight carboxylic acid in aqueous solution at a temperature of about 80° to about 120°C; or

for preparing an aminotriazole derivative of the general formula I wherein $A^-$ is an aminophosphonic acid anion or dianion of the formula II or III comprising

c) oxidizing the corresponding N-phosphonomethyl-aminodiacetic acid aminotriazole salt with hydrogen peroxide or a free oxygen containing gas in the presence of a catalyst.

European Patent Office

# EUROPEAN SEARCH REPORT

0146219

Application number

EP 84 30 6751

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-3 799 758 (J.E.FRANZ)<br>* Column 1, line 55 - column 4, line 14; column 14, lines 9-54 *<br>--- | 1-12 | C 07 F 9/38<br>C 07 F 9/30<br>C 07 D 249/14<br>A 01 N 57/18 |
| D,Y | EP-A-0 070 702 (MONSANTO COMP.)<br>* Page 9, example 1; claims *<br>--- | 1-12 | |
| Y | US-A-4 421 547 (M.P.PRISBYLLA)<br>* Whole document *<br>--- | 1-12 | |
| Y | CHEMICAL ABSTRACTS, Vol.97, No.13, 1982, page 195, abstract no. 105521b, COLUMBUS,OHIO.(US). G.BUDOI et al.:"Studies on the efficiency of some herbicides applied to a vineyard".<br>& Lucr. Stiint. - Inst. Agron. "Nicolae Balcescu", Bucuresti, Ser.B. 1981, 24, 75-7.<br>* Abstract *<br>----- | 1-12 | TECHNICAL FIELDS SEARCHED (Int. Cl.4)<br>C 07 F 9/00 |

The present search report has been drawn up for all claims

| Place of search | Date of completion of the search | Examiner |
|---|---|---|
| THE HAGUE | 26-02-1985 | BESLIER L. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document